(19) Europäisches Patentamt
European Patent Office
Office européen des brevets

(11) Publication number: 0 023 045 B1

(12) **EUROPEAN PATENT SPECIFICATION**

(45) Date of publication of patent specification: 11.04.84

(21) Application number: 80104287.0

(22) Date of filing: 21.07.80

(51) Int. Cl.³: C 07 D 499/64, C 07 D 501/20 //A61K31/43, A61K31/545, C07D487/14, C07D233/90

# (54) Imidazolecarboxylic acid derivatives of penicillins and cephalosporins.

(30) Priority: 20.07.79 JP 92940/79

(43) Date of publication of application: 28.01.81 Bulletin 81/4

(45) Publication of the grant of the patent: 11.04.84 Bulletin 84/15

(84) Designated Contracting States: CH DE FR GB IT LI NL

(56) References cited:
FR - A - 2 248 043
FR - A - 2 394 550
US - A - 3 320 240

The file contains technical information submitted after the application was filed and not included in this specification

(73) Proprietor: AJINOMOTO CO., INC.
5-8, Kyobashi 1-chome, Chuo-ku
Tokyo 104 (JP)

(72) Inventor: Yasuda, Naohiko
No. 2-36-2, Hairand
Yokosuka-shi, Kanagawa-ken (JP)
Inventor: Okutsu, Masaru
No. 3383-18, Fukuda
Yamato-shi, Kanagawa-ken (JP)
Inventor: Iwagami, Hisao
No. 2-20-8, Kannon
Kawasaki-ku, Kawasaki-shi Kanagawa-ken (JP)
Inventor: Nakamiya, Teruaki
No. 1-31-12, Seta
Setagaya-ku, Tokyo (JP)
Inventor: Takase, Ichiro
No. 2-20-8, Kannon
Kawasaki-ku, Kawasaki-shi Kanagawa-ken (JP)

(74) Representative: Schübel-Hopf, Ursula et al,
Strehl, Schübel-Hopf, Schulz Patentanwälte
Widenmayerstrasse 17
D-8000 München 22 (DE)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 023 045 B1

Imidazolecarboxylic acid derivatives of penicillins and cephalosporins

The present invention relates to novel imidazolecarboxylic acid derivatives, which may be used as antibiotics, particularly as agents for treating infectious diseases of human beings and animals caused by different bacteria, especially *Pseudomonas aeruginosa.*

According to the invention there has been found that some novel imidazole carboxylic acid derivatives of penicillin and cephalosporin show a marked antibacterial activity, particularly against Pseudomonas qeruginosa, as well as a broad antibacterial spectrum and therefore can be used as an antibiotic or as an intermediate for antibiotics.

IN US patent 3 320 240 there are described dicarboxylic acid derivatives of $\alpha$-aminopenicillin. Among the dicarboxylic acids which are useful for producing the derivative there are mentioned heterocyclic dicarboxylic acids, such as pyrazole dicarboxylic acids. The known compounds are antibacterial agents.

French published patent application 22 48 043 and 23 94 550 describe antibacterial penicillin derivatives comprising $\alpha$-aminopenicillin substituted by a carboxylic acid, which may be an imidazole carboxylic acid derivative. Among the stated specific compounds there is D-$\alpha$-(4-carboxyimidazole-5-carboxamido)-benzylpenicillin. The known compounds are used as antibacterial agents.

It has been found that the imidazole carboxylic acid derivatives according to the invention show a broader and different antibacterial spectrum than the most similar known compound.

According to the invention there is provided an imidazolecarboxylic acid derivative having the following general formula

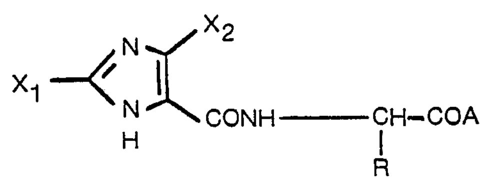

in which

$X_1$ is hydrogen, a hydroxyl, mercapto or an aralkylthio group,

$X_2$ is hydrogen, halogen, a nitro, amino-, acylamino, alkyloxycarbonyl, aralkyloxycarbonyl or aryloxycarbonyl group, a mono- or dialkyl-, mono- or diaralkyl-, mono- or diaryl-carbamoyl-group, an alkyl-, aralkyl or aryl group, wherein the carbon number of any of said alkyl, aralkyl or aryl groups is in the range of 1 to 10, or a morpholino-carbonyl group or a methanesulfonyl-amino group,

R is a phenyl group and A is an organic compound residue having the following general formula:

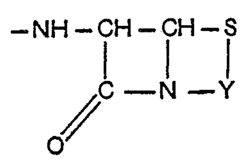 II

wherein Y is a bivalent residue having one of the following general formulae:

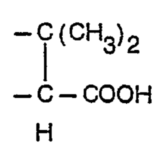 III and 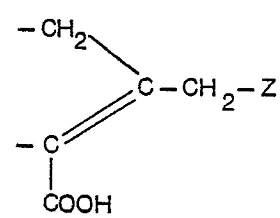 IV

wherein the carbon atom which carries the carboxyl group is bound to the nitrogen atom in the residue Y, and Z is a hydrogen atom, an acetoxy group, a carbamoyloxy group, a 5-(1-methyl-tetrazolyl) thio-, 5-[1-(2-sulfoethyl)tetrazolyl]-thio- or a 2-(1,3,4-thiadiazolyl) thio group, or a quaternary ammonium group, such as pyridinium, quinolinium or picolinium group, either unsubstituted or substituted with a 2-sulfoethyl- or carboxymethyl group.

The amino acid which forms the imidazolecarboxylic acid derivative of the present invention, which is derived from the residue containing R in the above described general formula is phenylglycine. Such amino acid is in the L-, D- or DL-form. In many cases the D-form is preferable in view of antibacterial activity. In the derivative of the general formula described above, the hydrogen atom of the

carboxyl group in the groups Y therein may be substituted by an atom or a group, for example, a metal atom such as sodium, potassium, calcium and aluminium, and a tertiary ammonium group such as triethylammonium, procain, dibenzylammonium, N-benzyl-β-phenethylammonium and an amino acid such as L-lysine. These salts or substituted products are included in the imidazolecarboxylic acid derivatives of the invention. Of course, in such case, pharmaceutically non-toxic substituent groups or salt forming ions respectively are employed.

It has been known that Penicillins and Cephalosporins having an amino group in α-position, shown by the general formula:

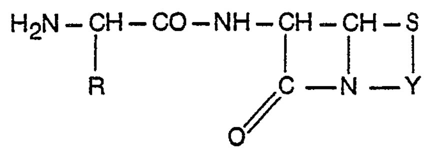

wherein R and Y are of the same meaning as described above, show antibacterial activity against not only gram positive bacteria but also gram negative bacteria. However, they show essentially no antibacterial activity against *Pseudomonas aeruginosa* which causes specifically serious infectious deseases.

The imidazolecarboxylic acid derivative of the present invention has an effective antibacterial activity against not only gram positive and gram negative bacteria but also *Pseudomonas aeruginosa*, and therefore has a very broad spectrum of antibacterial activity, and hence is a very valuable compound. The object compound of the present invention can be prepared by reacting α-amino-penicillins or cephalosporins shown by the following general formula:

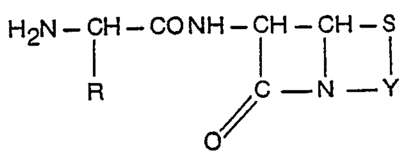

with a reactive derivative of an imidazolecarboxylic acid shown by the following general formula:

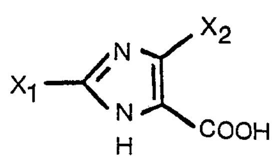

in a condensation reaction. In the above formulae, $X_1$, $X_2$, R and Y have the above described definition.

Examples of suitable reactive derivatives are the acid halide derivative, mixed acid anhydride, active amide and active ester. Particularly, the reactive derivative having the following general formula:

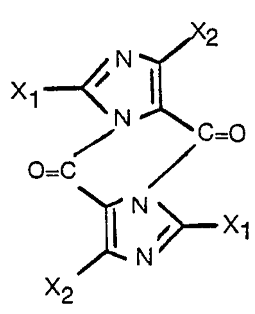

is employed frequently.

This derivative can be prepared by reacting an imidazolecarboxylic acid derivative of the formula

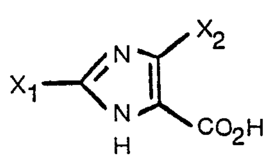

with a halogenating agent, such as thionyl chloride and phosphorous pentachloride.

Particularly, when $X_2$ represents a ester group shown by the formula, —$COOR_2$ wherein $R_2$ represents alkyl, aralkyl and aryl groups, or a morpholino-carbonyl group, such derivative can be prepared according to the following reaction scheme:

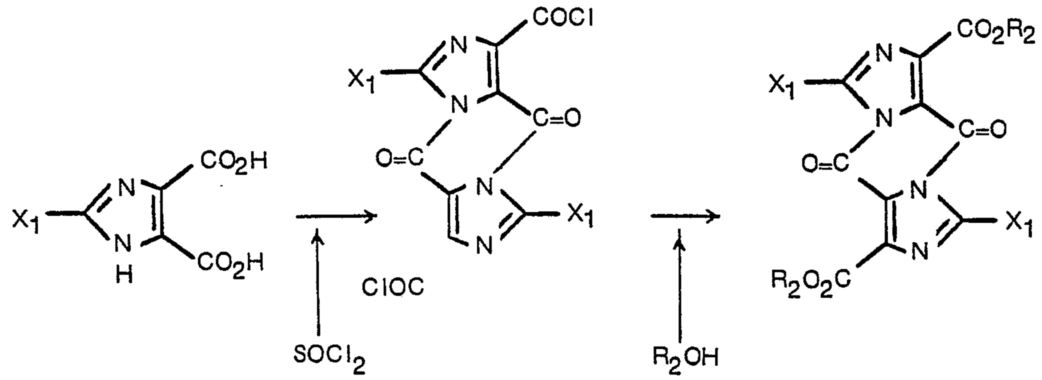


The reaction of the reactive derivative of the imidazoledicarboxylic acid as described above, with $\alpha$-aminopenicillins or $\alpha$-aminocephalosporins, is carried out in the presence of a base such as an alkali metal bicarbonate, alkali metal carbonate, trialkylamine, and pyridine. In the reaction, a solvent such as water, acetone, dioxane, acetonitrile, chloroform, methylenedichloride, tetrahydrofuran (THF), dimethyl formamide (DMF) can be employed. Hydrophilic organic solvents can be used in admixture with water. Desirably, the reaction is carried out at room temperature or a lower temperature.

2 Moles of $\alpha$-aminopenicillins or $\alpha$-aminocephalosporins are used for each mole of the reactive derivative (5,10-dioxo-5,10-dihydrodiimidazo [1,5a, 1′,5′ d] pyrazine-1,6-dicarboxylic acid ester).

For reacting with the reactive derivative, $\alpha$-aminopenicillins or $\alpha$-aminocephalosporins shown by the general formula as described above in which the carboxyl group in the Y is esterified, can be used. For example, t-butyl esters, benzyl esters, silyl esters, trichloroethyl esters, diphenylmethyl esters, and the like, can be used in the reaction. Subsequent to the reaction, the ester groups can be removed to regenerate the free carboxylic acid groups. The object compound, modified $\alpha$-aminocephalosporins shown by the general formula described above, wherein A has a cephalosporin backbone and further Z is a heteroaromatic thio group or quaternary ammonium group can be obtained by synthesizing the corresponding cephalosporins wherein Z is an acetoxy group and then replacing the acetoxy group by the heteroaromatic thio group or quaternary ammonium group by conventional methods.

Modified $\alpha$-aminocephalosporins shown by the general formula described above, wherein $X_2$ is a group such as an amino group can be obtained by synthesizing the corresponding cephalosporins wherein $X_2$ is a nitro group and then transforming the nitro group to the amino group by catalytic reduction. Further, by acylating the thus obtained compound wherein $X_2$ is a amino group by an acid halide or the like, the object compound wherein $X_2$ is a acylamino group can be obtained. The reaction products of the modification reaction can be isolated in pure form by known procedures, for example by extraction, column chromatography, recrystallization and the like.

The thus obtained imidazolecarboxylic acid derivative is converted to non-toxic salts thereof such as alkalimetal salts, ammonium salts or organic base salts. These salts are desirable, for example, for preparations since they are soluble in water.

The present invention is explained precisely in the following Examples.

Example 1

4-nitroimidazole-5-carboxylic acid (1.57 g, 10 mM) was suspended in dry benzene (20 ml), and thereto DMF (3 drops) and then thionyl chloride (4 ml) were added. The thus obtained mixture was refluxed at a temperature of 80°C while stirring for 3 hours. After completion of the reaction, the reaction mixture was concentrated to yield a solid material. To the thus obtained material, dry benzene (10 ml) was added and the mixture was again concentrated to yield a solid material. The remaining material was washed with a mixture of benzene (10 ml) and petroleum ether (10 ml) and then collected by filtration, and dried to give the reactive derivative, 1,6-dinitro-5,10-dioxo-5,10-dihydrodiimidazo-[1,5a, 1′,5′d] pyrazine (1.3 g, 4.7 mM). Yield: 94%.

I.R. spectrum (Nujol):

1755 $cm^{-1}$, 1550 $cm^{-1}$, 1285 $cm^{-1}$, 1230 $cm^{-1}$

On the other hand, anhydrous D(—)-$\alpha$-aminobenzylpenicillin (2.8 g, 8 mM) was suspended in dichloromethane (30 ml). To this mixture, triethylamine (4.5 ml) was added and a homogeneous

solution was obtained. To this solution the reactive derivative which had been previously synthesized, was added while stirring under cooling. The mixture was stirred under cooling for two hours, and then was stirred overnight at room temperature. Insoluble material was removed by filtration from the reaction solution and the thus obtained solution was concentrated under reduced pressure at 30°C or lower to yield a solid material. The thus obtained solid material was dissolved in water (50 ml) and then ethyl acetate (50 ml) was added thereto to form two phases. The ethyl acetate phase was removed and to the obtained aqueous phase ethyl acetate (80 ml) was added and then 6% aqueous HCl solution was added while stirring to adjust the aqueous phase to pH 1.5. The precipitated insoluble material was removed by filtration and a solution having two phases was obtained. From the aqueous phase, organic material was extracted with ethyl acetate (70 ml), once more. All the obtained ethyl acetate phases were combined and dried over anhydrous magnesium sulfate. The ethyl acetate solution was concentrated at 30°C or lower and to the thus obtained solid material ether was added to produce a powder. The powder was collected by filtration and dried to give the desired product, D-α-(4-nitro-imidazole-5-carbonylamino benzyl penicillin (2.6 g, yield: 67%).

The thus obtained product was dissolved in a mixture (30 ml) of methanol and ethyl acetate (v/v=1/1) and to the solution 2-ethylhexane carboxylic acid sodium salt n-butanol solution (2 M/l) (3.3 ml) was added. The mixture was stirred for 10 minutes, and to this mixture ether (70 ml) was added dropwise to precipitate the solid material. This material was cooled in the refrigerator, and the thus obtained solid material was obtained by filtration and dried to give the desired product, D-α-(4-nitro-imidazole-5-carbonylamino) benzylpenicillin sodium salt (3.0 g).

I.R. spectrum (Nujol):

$\nu_{co}$ (β-lactam) = 1770 $cm^{-1}$

Example 2—9

The products shown by the following formula were produced in the same manner as described in Example 1. The results

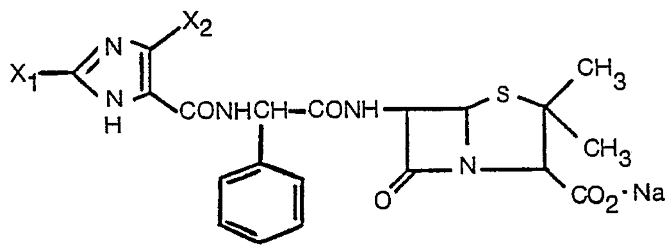

are listed in Table 1.

TABLE 1

| Example No. | Products of the Present Invention X1 | X2 | I.R. Spectrum $\nu_{co}$ (β-lacton) (Nujol) | Yield |
|---|---|---|---|---|
| 2 | –H | –H | 1780 $cm^{-1}$ | 11% |
| 3 | –H | –Cl | 1780 | 23 |
| 4 | –H | $-NHSO_2CH_3$ | 1780 | 19 |
| 5 | –H | $-CH_3$ | 1780 | 31 |
| 6 | –H | $-NHCOCH_3$ | 1770 | 56 |
| 7 | 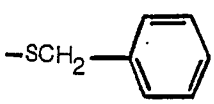 | $-COOC_2H_5$ | 1780 | 62 |
| 8 | –SH | $-CH_3$ | 1775 | 43 |
| 9 | –OH | –H | 1770 | 28 |

Example 10

30% Pd-$BaCO_3$ (4.8 g) was suspended in water (60 ml) and was activated by stirring the mixture under 30 atmospheric pressure of $H_2$ in a autoclave for 1 hour. To this mixture D-$\alpha$-(4-nitroimidazole-5-carbonylamino) benzylpenicillin sodium salt (1.5 g, 3 mM) as obtained in Example 1 in water (60 ml), solution was added. The reduction reaction was carried by stirring the thus obtained mixture under atmospheric pressure of $H_2$ at room temperature for 1 hour. After completion of the reaction, the catalyst was removed by filtration, and the pH of the aqueous solution was adjusted to 7 and then thus obtained mixture was washed with ethyl acetate (200 ml). To the aqueous phase ethyl acetate (200 ml) was added and then 6% aqueous HCl solution was added while stirring to adjust the aqueous phase to pH 1.5.

The ethyl acetate phase was separated and then from the aqueous phase, organic material was extracted with ethyl acetate (200 ml), once more. All the obtained ethyl acetate phases were combined, washed with water and dried over anhydrous magnesium sulfate.

The ethyl acetate solution was concentrated at 30°C or less and to the thus obtained residue ether was added to produce a powder. The thus obtained powder was collected by filtration and dried to give the desired product, D-$\alpha$-(4-aminoimidazole-5-carbonylamino) benzylpenicillin (1.0 g, yield: 73%).

I.R. spectrum (Nujol):
$\nu_{co}$ ($\beta$-lactam) = 1775 $cm^{-1}$

Example 11

7-$\beta$-[D(—)-$\alpha$-aminophenylacetylamino] cephalosporanic acid (1.8 g. 4.5 mM) was suspended in dichloromethane (20 ml), and to this mixture triethylamine (2.5 ml) was added to give a homogeneous solution. To this solution, 1,6-dinitro-5,10-dioxo-5,10-dihydroiimidazo[1.5a, 1',5'd] pyrazine (0.8 g, 2.9 mM) produced in the same manner as in Example 1, was added while stirring under cooling.

Hereinafter in the same manner as in Example 1, the desired product, 7-$\beta$-[D(—)-$\alpha$-(4-nitroimidazole-5-carbonylamino)phenylacetylamino]-cephalosporanic acid sodium salt (1.3 g, yield: 53%), was obtained.

I.R. spectrum (Nujol):
$\nu_{co}$ ($\beta$-lactam) = 1785 $cm^{-1}$
$\nu_{co}$ (—$OCOCH_3$) = 1745 $cm^{-1}$

N.M.R. spectrum (Solvent: $D_2O$)
$\delta$ 2.06 (S. 3H) (—$OCOCH_3$)
3.28 (m. 2H) (>$CH_2$, 2-position)
4.95 (d, 1H) (—H, 6-position)
5.60 (S, 1H) ( 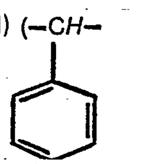 )
5.66 (d, 1H) (—H, 7-position)
7.33 (b, S, 5H) ( 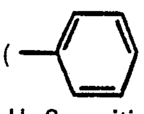 )
7.82 (S, 1H) (—H, 2-position of imidazole ring)

Example 12

Imidazoledicarboxylic acid (7.8 g, 0.05 M) was suspended in dry benzene (100 ml), and thereto DMF (4 ml) and then thionyl chloride (30 ml) were added. The thus obtained mixture was refluxed at a temperature of 85°C while stirring for six hours. After completion of the reaction, the reaction solution was concentrated to give a solid material. To the solid material, dry benzene (50 ml) was added and the thus obtained mixture was concentrated to give a solid material, once more. To the remaining material, benzene (50 ml) was added and the mixture was stirred at room temperature for 30 minutes. The insoluble material was obtained by filtration, washed with benzene, and dried under reduced pressure to give the desired product, 5,10-dioxo-5,10-dihydrodiimidazo[1,5a, 1',5'd]pyrazine-1,6-dicarboxylic acid dichloride (7.0 g, yield: 89%).

The thus obtained acid chloride derivative (7.0 g) was suspended in ethanol (150 ml) and the thus obtained mixture was stirred at room temperature overnight. The insoluble solid material was obtained by filtration, washed with ethanol and then ether, and dried under reduced pressure to give 5,10-dioxo-5,10-dihydrodiimidazo[1,5a,1',5' d] pyrazine-1,6-dicarboxylic acid diethyl ester (7.0 g, yield: 94%).

I.R. Spectrum (Nujol):

$1760\ cm^{-1}$, $1745\ cm^{-1}$, $1725\ cm^{-1}$ 1570, $cm^{-1}$, $1255\ cm^{-1}$, $1190\ cm^{-1}$, $1140\ cm^{-1}$, 1010 $cm^{-1}$, $925\ cm^{-1}$, $743\ cm^{-1}$

Elemental analysis:

Found C 49.60%, H 3.61%, N 16.52%

Calcd. as $C_{14}H_{12}N_4O_6$

C 50.60%, H 3.64%, N 16.86%

On the other hand, 7-$\beta$-[D(—)-$\alpha$-aminophenylacetylamino] cephalosporanic acid (3.2 g, 8 mM) was suspended in dichloromethane (50 ml), and to this mixture triethylamine (3 ml) was added to give homogeneous solution. To this solution, 5,10-dioxo-5,10-dihydrodiimidazo [1,5a, 1',5'd] pyrazine-1,6-dicarboxylic acid diethyl ester (1.3 g, 4 mM) was added while stirring under cooling. The mixture was stirred at room temperature overnight. The thus obtained mixture was concentrated to give solid material. To this material, water (30 ml) was added and stirred to give a homogeneous solution. The solution was adjusted to pH 7.5 by adding 6% aqueous HCl solution, stirred for 10 minutes, and then washed with ethyl acetate (50 ml). The aqueous phase was adjusted to pH 2 by adding 6% aqueous HCl solution, and then stirred for 20 minutes. The precipitated crystals were obtained by filtration, washed with water, and then dried under reduced pressure at 40°C. The thus obtained solid material was suspended in a mixture (300 ml) of ethyl acetate and ethanol (volume ratio: 1/1). The mixture was stirred at 40°C for 20 minutes. An insoluble material was obtained and then the organic phase was concentrated under reduced pressure to a volume of 50 ml. To this mixture, ether (500 ml) was added and the thus obtained mixture was kept in a refrigerator overnight.

The precipitated crystals were obtained by filtration, washed with petroleum ether, and dried to give the desired object product, 7-$\beta$-[D(—)-$\alpha$-(4-ethoxycarbonylimidazole-5-carbonylamino)-phenyl-acetylamino]cephalosporanic acid (3.4 g, yield: 75%).

I.R. spertrum (Nujol):

$\nu_{co}$ ($\beta$-lactam) = $1775\ cm^{-1}$'

$\nu_{co}$ (—$CO_2Et$) = $1730\ cm^{-1}$

Example 13

Anhydrous D(—)-$\alpha$-aminobenzylpenicillin (2.8 g, 8 mM) was reacted with 5,10-dioxo-5,10-dihydrodiimidazo[1,5a,1',5'd]pyrazine-1,6-dicarboxylic acid diethyl ester (1.3 g, 4 mM) to give D-$\alpha$-(4-ethoxycarbonylimidazole-5-carbonylamino) benzylpenicillin (3.0 g, yield: 73%) in the same manner as in Example 12.

I.R. spectrum (Nujol):

$\nu_{co}$ ($\beta$-lactam) = $1770\ cm^{-1}$

$\nu_{co}$ (—$CO_2Et$) = $1730\ cm^{-1}$

N.M.R. spectrum ($d_6$-DMSO)

$\delta$ 1.33 (t, 3H) (—$COOCH_2C\underline{H}_3$)

1.43 (s, 3H)
1.57 (s, 3H) } ( 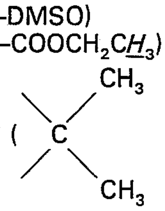 )

4.20 (s, 1H) (—H, 3-position)

4.37 (q, 2H) (—$COOC\underline{H}_2$—$CH_3$)

5.30~5.70 (m, 2H) (—H, 5-position; —H, 6-position)

5.89 (d, 1H) ( 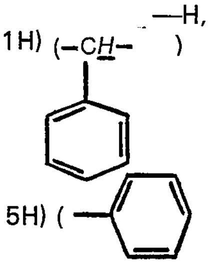 )

7.40 (m, 5H) ( )

7.83 (s, 1H) (—H, 2-position of imidazole)

Example 14

5,10-dioxo-5,10-dihydrodiimidazo[1,5a,1',5'd]pyrazine-1,6-dicarboxylic acid dichloride (7.0 g)

obtained in Example 12, was suspended in water (150 ml) and the thus obtained mixture was stirred at room temperature overnight. An insoluble solid material was obtained by filtration, washed with water, a small amount of THF, and ether successively, and dried under reduced pressure to give the desired product, 5,10-dioxo-5,10-dihydroimidazo[1,5a,1',5'd]pyrazine-1,6-dicarboxylic acid 2 hydrate (7.0 g, yield: 100%). Melting point: 248°C (dec.).

I.R. spectrum:
$3500\ cm^{-1}$, $1750\ cm^{-1}$, $1710\ cm^{-1}$, $1255\ cm^{-1}$, $930\ cm^{-1}$

Elemental analysis:
Found C 38.65%, H 2.40%, N 18.02%
Calcd. for $C_{10}H_4N_4O_6 \cdot 2H_2O$
C 38.47%, H 2.58%, N 17.95%

Isobutylamine (6.6 g, 90 mM) was dissolved in dichloromethane (200 ml), and thereto triethylamine (22 ml) and then the above mentioned 5,10-dioxo-5,10-dihydrodiimidazo[1,5a,1',5'd]-pyrazine-1,6-dicarboxylic acid 2 hydrate (9.4 g, 30 mM) were added. The thus obtained mixture was reacted while stirring and being heated at 40°C for 5 hours. An insoluble material was removed by filtration, and the remaining filtrate was concentrated. The remaining residue was dissolved in water (150 ml) to give an aqueous solution of pH 10. The solution was twice extracted with ethyl acetate (150 ml) and then the aqueous phase was adjusted to pH 2 with 2N—HCl. The thus obtained mixture was kept in a refrigerator overnight. The precipitated material was obtained by filtration and dried under reduced pressure and heating. The thus obtained material was dissolved and heated at 50°C in THF (300 ml) to dissolve the material. An insoluble material was removed by filtration. The obtained filtrate was concentrated and thereto ether was added to produce crystals. The thus obtained mixture was cooled overnight. The precipitated crystals were obtained by filtration, washed with petroleum ether, and dried under reduced pressure to give 4-isobutylcarbamoylimidazole-5-carboxylic acid (9.2 g, yield: 73%)

N.M.R. spectrum ($d_6$-DMSO)
$\delta$ 0.90 (d, 6H) (—CH($C\underline{H}_3)_2$)
1.98 (m, 1H) (—$CH_2$—$C\underline{H}$<)
3.20 (dd, 2H (—$C\underline{H}_2$—CH<)
8.08 (s, 1H) (—H, 2-position of imidazole)
9.33 (t, 1H) (—CON$\underline{H}$$CH_2$—)

The thus obtained 4-isobutylcarbamoylimidazole-5-carboxylic acid (2.1 g, 10 mM) was suspended in dry benzene (30 ml), and thereto DMF (5 drops) and then thionyl chloride (6 ml) were added. The thus obtained mixture was refluxed at 80°C while stirring for 4 hours. After completion of the reaction the reaction mixture was concentrated under reduced pressure to give a solid material.

To the thus obtained material, dry benzene (30 ml) was added, and the thus obtained solution was again concentrated to give a solid material, 1,6-isobutylcarbamoyl-5,10-dioxo-5,10-dihydro-diimidazo[1,5a,1',5'd] pyrazine.

On the other hand, 7-$\beta$-[D(—)-$\alpha$-aminophenylacetylamino] cephalosporanic acid (4.0 g, 10 mM) was suspended in dichloromethane (75 ml), and thereto triethylamine (4.5 ml) was added to give a homogeneous solution.

The thus obtained solution was added to the suspension of the reactive derivative in dichloromethane (30 ml) which had been previously prepared while stirring and being cooled. The mixture was stirred under cooling for 2 hours and then stirred at room temperature overnight.

From the reaction solution, an insoluble material was removed by filtration and the filtrate was concentrated under reduced pressure at 30°C or less to give a solid material. The thus obtained solid material was dissolved in water (50 ml), and ethyl acetate (50 ml) was added thereto to yield two phases.

The ethyl acetate phase was removed and to the obtained aqueous phase ethyl acetate (80 ml) was added and then 6% aqueous HCl solution was added while stirring to adjust the aqueous phase to pH 1.5. The precipitated insoluble material was removed by filtration and a solution having two phases was obtained from the aqueous phase, organic material was extracted with ethyl acetate (80 ml), once more. The obtained ethyl acetate phase were combined and dried over anhydrous magnesium sulfate. The ethyl acetate solution was concentrated at 30°C or lower and to the thus obtained residue ether was added to produce a powder. The thus obtained mixture was stood in a refrigerator overnight. The powder was collected by filtration and dried to give the desired product, 7-$\beta$-[D(—)-$\alpha$-(4-isobutyl-carbamoylimidazole-5-carbonylamino)-phenylacetylamino] cephalosporanic acid (2.2 g, yield: 37%).

I.R. spectrum (Nujol):
$\nu_{co}$ ($\beta$-lactam) = 1780 $cm^{-1}$
$\nu_{co}$ ($-OCOCH_3$) = 1730 $cm^{-1}$

N.M.R. spectrum ($d_6$-DMSO):
$\delta$ 0.90 (d, 6H) ($-CH(C\underline{H}_3)_2$)
2.00 (s, 3H) ($-OCO\cdot C\underline{H}_3$)
whose signal overlaps one of "$-C\underline{H}(CH_3)_2-$"
3.10 (m, 2H) ($-C\underline{H}_2-CH<$)
3.45 (m, 2H) ($>CH_2$, 2-position)
4.80 (q, 2H) ($>CH_2$, 3-position)
5.00 (d, 1H) (—H, 6-position)
5.75 (m, 2H) ($-C\underline{H}-$ + —H, 7-position)

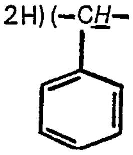

7.30 (b, s, 5H) ( 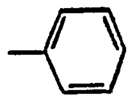 )
7.82 (s, 1H) (—H, 2-position of imidazole)

Example 15

5,10-dioxo-5,10-dihydrodiimidazo[1,5a,1',5'd]pyrazine-1,6-dicarboxylic acid·2 hydrate was reacted with morpholine in the same manner as in Example 14 to give 4-morpholinocarbonylimidazole-5-carboxylic acid (Yield: 16%).

N.M.R. spectrum ($d_6$-DMSO)
$\delta$ 3.70 (b, s, 8H) (— 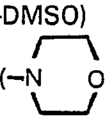 )
8.00 (s, 1H) (—H, 2-position of imidazole)

Thus obtained product was reacted with 7-$\beta$-[D(—)-$\alpha$-aminophenylacetylamino] cephalosporanic acid in the same manner as Example 14 to give the desired product, 7-$\beta$-[D(—)-$\alpha$-(4-morpholinocarbonylimidazole-5-carbonylamino)-phenylacetyl amino] cephalosporanic acid (Yield: 21%).

Example 16

4-Diethylcarbamoylimidazole-5-carboxylic acid was obtained (yield: 12%) by reacting 5,10-dioxo-5,10-dihydrodiimidazo[1,5a,1',5'd] pyrazine-1,6-dicarboxylic acid · 2 hydrate with diethylamine in the same manner as in Example 14.

N.M.R. spectrum ($d_6$-DMSO):
$\delta$ 1.22 (t, 6H) ($-CH_2C\underline{H}_3$)
3.90 (q, 4H) ($-C\underline{H}_2CH_3$)
7.90 (s, 1H) (—H, 2-position of imidazole)

This product was reacted with 7-$\beta$-[D(—)-$\alpha$-aminophenylacetylamino] cephalosporanic acid in the same manner as in Example 14 to give the desired product, 7-$\beta$-[D(—)-$\alpha$-(4-diethylcarbamoylimidazole-5-carbonylamino)-phenylacetylamino] cephalosporanic acid (yield: 28%).

Example 17

7-$\beta$-[D(—)-$\alpha$-(4-ethoxycarbonylimidazole-5-carbonylamino)-phenylacetylamino] cephalosporanic acid (1.7 g, 3 mM) as produced in Example 12 and 4-pyridine ethane sulfonic acid (1.1 g, 6 mM) were suspended in water (15 ml), and thereto a 2N—NaOH aqueous solution was added dropwise while stirring to adjust the pH of the solution to 7. In this manner a homogeneous solution was obtained. Sodium iodide (12 g) was added thereto, and the thus obtained mixture was reacted while stirring at 70°C for 2 hours. After completion of the reaction, the reaction solution was treated with Amberlite (Trade Mark) XAD—2 produced by Rohm and Hass Co., Ltd. (700 ml) for absorption and excess sodium iodide and 4-pyridine ethane sulfonic acid were eluted with water. By elution with a mixture of water and methanol (1:1), the desired product was eluted. Fractions of the desired product were collected, and therefrom methanol was removed under reduced pressure. By freeze-drying the remaining solution, the desired product, 7 - $\beta$ - [D(—) - $\alpha$ - (4 - ethoxycarbonylimidazole - 5 - carbonylamino) - phenylacetylamino - 3 - (4 - β - sulfoethylpyridinium) - methyl - 3 - cephem - 4 - carboxylic acid sodium salt was obtained (0.59 g, yield: 26%)

I.R. spectrum (Nujol):
$\nu_{co}$ (β-lactam) = 1760 $cm^{-1}$
$\nu_{co}$ ($—SO_3H$) = 1210 $cm^{-1}$, 1040 $cm^{-1}$

N.M.R. spectrum ($D_2O$):
δ 1.33 (t, 3H) ($—CO—OCH_2C\underline{H}_3$)
3.20 (m, 2H) ($>CH_2$, 2-position)
3.34 (s, 4H) (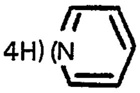 $—C\underline{H}_2C\underline{H}_2SO_3$)
4.20 (t, 2H) ($—C—OC\underline{H}_2CH_3$)
5.05 (d, 1H) (—H, 6-position)
5.40 (m, 2H) ($—CH_2—$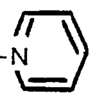)
5.62 (s, 1H) ($—C\underline{H}—$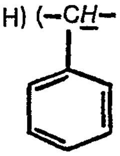)
5.75 (d, 1H) (—H, 7-position)
7.40 (b, s, 5H) (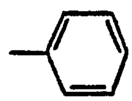)
7.85 (s, 1H) (—H, 2-position of imidazole)
7.97 (d, 2H) (—H, 3 and 5-position of pyridine ring)
8.84 (d, 2H) (—H, 2 and 6-position of pyridine ring)

## Example 18

7-β-[D(—)-α-(4-ethoxycarbonylimidazole-5-carbonylamino)-phenylacetylamino] cephalosporanic acid (1.5 g, 2.6 mM) as produced in Example 12 and 1-methyl-5-mercaptotetrazole (0.33 g, 2.9 mM) were suspended in phosphate buffer solution (PH 6.4) (25 ml) and then 2N—NaOH aqueous solution was added dropwise to adjust the solution to pH 6.4, to obtain an homogeneous solution. This solution was reacted while stirring at 60°C for 24 hours. After 5 hours' reaction the solution was adjusted to pH 6.4 with 2N—NaOH aqueous solution. After completion of the reaction water (45 ml) was further added to the reaction mixture and then the solution was adjusted to pH 7 with 2N—NaOH aqueous solution.

An insoluble material was removed by filtration, and the thus obtained filtrate was washed with ethyl acetate (60 ml). To the aqueous solution, ethyl acetate (100 ml) was added and then 6% aqueous HCl solution while stirring to adjust the aqueous phase to pH 2.0. An insoluble material was removed by filtration and a solution having two phases was obtained. From the aqueous phase, organic material was extracted with ethyl acetate (100 ml), once more. All the obtained ethyl acetate phases were combined washed with water, and then dried over anhydrous magnesium sulfate.

The ethyl acetate solution was concentrated and to the thus obtained solid material, ether was added to produce a powder. The thus obtained mixture was cooled in a refrigerator, and then the thus produced powder was collected by filtration and dried to give the desired product. 7 - β - [D(—) - α - (4 - ethoxycarbonylimidazole - 5 - carbonylamino) - phenylacetylamino] - 3 - (1 - methyl - 1H - tetrazol - 5 - yl)thiomethyl - 3 - cephem - 4 - carboxylic acid (0.7 g, yield: 47%).

I.R. spectrum (Nujol):
$\nu_{co}$ (β-lactam) = 1770 $cm^{-1}$

N.M.R. spectrum ($d_6$-DMSO)
δ 1.35 (t, 3H) ($—CO \cdot OCH_2C\underline{H}_3$)
3.60 (m, 2H) ($>CH_2$, 2-position)
3.93 (s, 3H) ($—CH_3$ in tetrazole)
4.30 (m, 4H) ($—CO \cdot OC\underline{H}_2CH_3$ + $—CH_2—S—$, 3-position)
4.96 (d, 1H) (—H, 6-position)

5.50~5.90 (m, 2H) (—H, 7-position + —CH— )

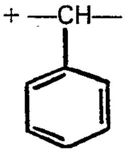

7.30 (m, 5H) ( 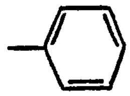 )

7.83 (s, 1H) (—H, 2-position of imidazole)

Examples 19 to 24

The compound shown by the following formula:

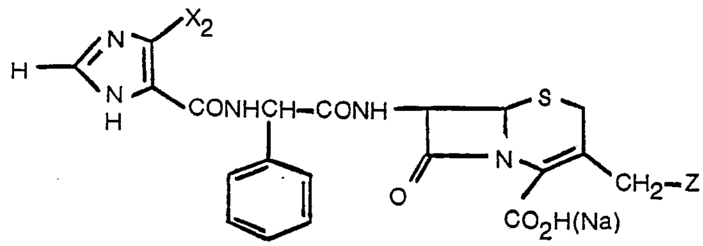

was produced in the same manners as in Examples 17 and 18, by using the compound produced in Examples 14—16 as starting material. The results were shown in Table 2.

TABLE 2

| Example No. | Compound of the Present Invention | | I.R. Spectrum (Nujol) ($cm^{-1}$) | Yield (%) |
|---|---|---|---|---|
| | $X_2$ | Z | | |
| 19 | $-CONHCH_2CH(CH_3)_2$ | 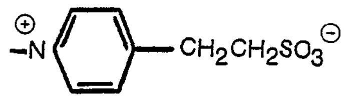 | 1760<br>1640<br>1215<br>1040 | 23% |
| 20 | ,, | 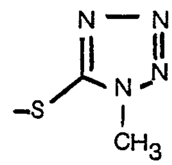 | 1760 | 53% |
| 21 | 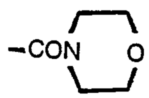 | 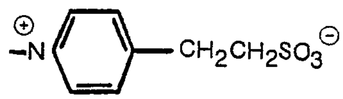 | 1760<br>1640<br>1210<br>1040 | 18% |
| 22 | ,, | 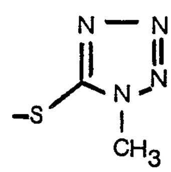 | 1765 | 34% |
| 23 | 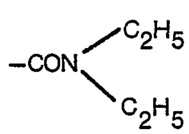 | 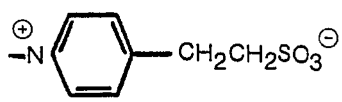 | 1760<br>1640<br>1210<br>1040 | 21% |
| 24 | ,, | 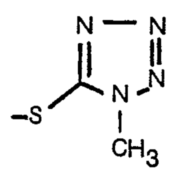 | 1760 | 37% |

Examples 25 to 29

5,10-dioxo-5,10-dihydrodiimidazo[1,5a,1',5'd]pyrazine-1,6-dicarboxylic acid dichloride was reacted with alcohol shown by the general formula R'OH, in the same manner as in Example 12, to produce the compound shown by the following formula:

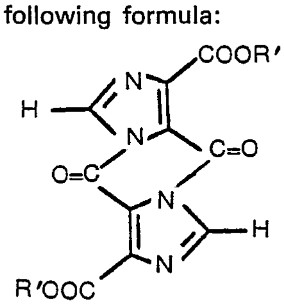

The results were shown in Table 3.

TABLE 3

| Example No. | Compound R′ | N.M.R. spectrum ($d_6$–DMSO) | Yield (%) |
|---|---|---|---|
| 25 | $-CH_3$ | δ: 3.93(6H,S), 8.97(2H,S) | 93.8 |
| 26 | 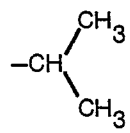 | δ: 1.37(12H,d), 5.23(2H,Sept), 8.93(2H,S) | 84.9 |
| 27 | $-CH_2CH_2CH_2CH_3$ | δ: 0.93(6H,t), 1.53(4H,m), 1.70(4H,m), 4.33(4H,t), 8.93(2H,s) | 49.8 |
| 28 | 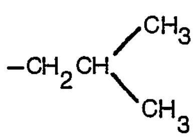 | δ: 1.00(12H,d), 2.07(2H,non) 4.13(4H,d), 8.97(2H,S) | 76.6 |
| 29 | 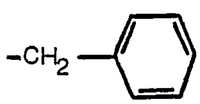 | δ: 5.43(4H,S), 7.47(10H,m), 8.93(2H,S) | 69.9 |

The obtained compound was reacted with anhydrous D(—)-α-aminobenzylpenicillin in the same manner as described in Examples 12 and 13, to produce the compound shown by the following formula:

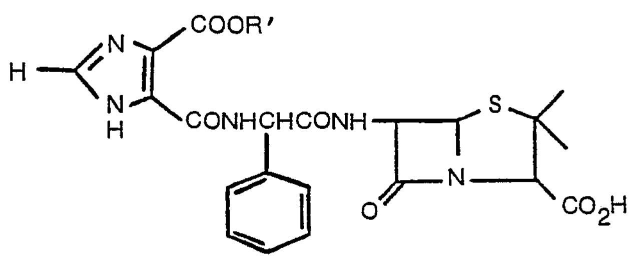

TABLE 4

| Example No. | Compound R′ | N.M.R. spectrum ($d_6$–DMSO) | Yield (%) |
|---|---|---|---|
| 25 | $-CH_3$ | δ: 1.43(3H,S), 1.57(3H,S), 3.90(3H,S), 4.22(1H,S), 5.30–5.80(2H), 6.00(1H), 7.10–7.70(5H), 7.87(1H,S) | 25.0 |
| 26 | 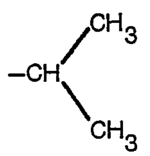 | δ: 1.33(6H,d), 1.42(3H,S), 1.57(3H,S), 4.20(1H,S), 5.20(1H,m), 5.27–5.80(2H), 5.97(1H), 7.10–7.70(5H), 7.83(1H,S) | 17.4 |
| 27 | $-CH_2CH_2CH_2CH_3$ | δ: 0.93(3H,t), 1.27–1.93(4H,m), 1.43(3H,S), 1.57(3H,S), 4.20(1H,S), 4.33(2H,t), 5.33–5.67(2H), 6.00(1H), 7.07–7.73(5H), 7.83(1H,S) | 29.0 |
| 28 | 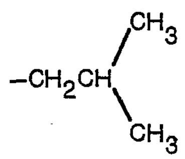 | δ: 1.03(6H,d), 1.43(3H,S), 1.57(3H,S), 1.70–2.33(1H), 4.13(2H,d), 4.20(1H,S), 5.33–5.67(2H), 6.00(1H), 7.10–7.77(5H), 7.87(1H,S) | 23.0 |
| 29 | 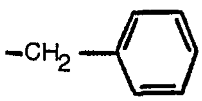 | δ: 1.43(3H,S), 1.57(3H,S), 4.20(1H,S), 5.27–5.80(2H), 5.40(2H,S), 6.00(1H), 7.07–7.73(10H), 7.87(1H,S) | 19.4 |

The imidazolecarboxylic acid derivatives of the present invention have a good antibacterial activity with a wide spectrum of activity, particularly against *Pseudomonas aeruginosa.*

For the compounds produced in the Examples described above, the anti-bacterial activity was determined. Some of Comparative Minimum Inhibitory Concentrations (MIC) (the minimum concentration of the compound in micrograms per milliliter required to inhibit the growth of the test organism in a culture) obtained by using *Pseudomonas aeruginosa* AJ 2116 as the test organism, are presented in Table 5.

TABLE 5

| Sample Example No. | MIC ($\mu$g/ml) |
|---|---|
| 1 | 12.5 |
| 3 | 25 |
| 8 | 25 |
| 13 | 25 |
| Carbenicillin | 100 |
| Ampicillin | More than 500 |

The imidazolecarboxylic acid derivatives of the present invention have a good oral absorption, as is evident from Table 6.

TABLE 6

Concentration in Blood ($\mu$g/ml)

Mouse (ICR 5W ♂)

Oral Administration: 50 mg/kg

| Sample / Time (minutes) | 30 | 60 | 90 |
|---|---|---|---|
| Compound produced in Example 13 | 340 | 97 | 40 |
| Ampicillin | 81 | 306 | 41 |

As is evident from the results, it is understood that the imidazolecarboxylic acid derivatives of the present invention have a good antibacterial activity and oral absorption, and therefore, can be practically used as medicines for human beings and the other animals, particularly for oral administration.

**Claims**

1. An imidazolecarboxylic acid derivative having the following general formula

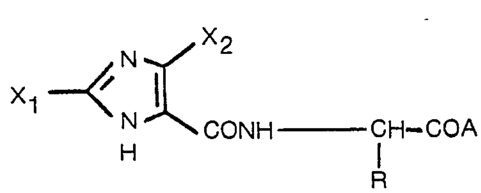

I

in which

$X_1$ is hydrogen, a hydroxyl, mercapto or an aralkylthio group,

$X_2$ is hydrogen, halogen, a nitro, amino, acylamino, alkyloxycarbonyl, aralkyloxycarbonyl or aryloxycarbonyl group, a mono- or dialkyl-, mono- or diaralkyl-, mono- or diaryl-carbamoyl-group, an alkyl, aralkyl or aryl group, wherein the carbon number of any of said alkyl, aralkyl or aryl groups is in the

range of 1 to 10, or a morpholino-carbonyl group or a methanesulfonyl-amino group,

R is a phenyl group and A is an organic compound residue having the following general formula:

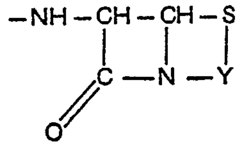

II

wherein Y is a bivalent residue having one of the following general formula:

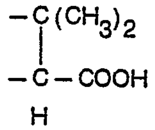

III and

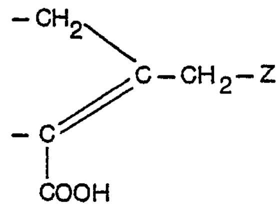

IV

wherein the carbon atom which carries the carboxyl group is bound to the nitrogen atom in the residue Y, and Z is a hydrogen atom, an acetoxy group, a carbamoyloxy group, a 5-(1-methyl-tetrazolyl) thio-, 5-[1-(2-sulfoethyl)tetrazolyl]-thio- or a 2-(1,3,4-thiadiazolyl) thio group, or a quaternary ammonium group, such as pyridinium, quinolinium or picolinium group, either unsubstituted or substituted with a 2-sulfoethyl- or carboxymethyl group.

2. An imidazolecarboxylic acid derivative according to claim 1, wherein the amino acid which forms said imidazolecarboxylic acid derivative is in the D-form.

3. An imidazolecarboxylic acid derivative according to claim 1 or 2, wherein said imidazolecarboxylic acid derivative is the salt form.

**Patentansprüche**

1. Imidazolcarbonsäurederivat der folgenden allgemeinen Formel

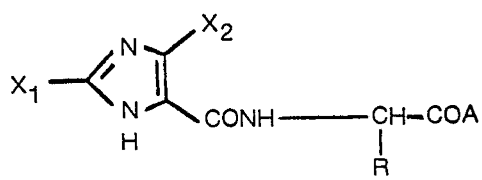

I

in der $X_1$ Wasserstoff, eine Hydroxyl-, Mercapto- oder eine Aralkylthiogruppe, $X_2$ Wasserstoff, Halogen, eine Nitro-, Amino-, Acylamino-, Alkyloxycarbonyl-, Aralkyloxycarbonyl- oder Aryloxycarbonylgruppe, eine Mono- oder Dialkyl-, Mono- oder Diaralkyl-, Mono- oder Diaryl-carbamoylgruppe, eine Alkyl-, Aralkyl- oder Arylgruppe, wobei die Kohlenstoffzahl jeder dieser Alkyl-, Aralkyl- oder Arylgruppen im Bereich von 1 bis 10 liegt, oder eine Morpholino-carbonylgruppe oder eine Methansulfonylaminogruppe darstellt, R eine Phenylgruppe ist und A den Rest einer organischen Verbindung der folgenden allgemeinen Formel

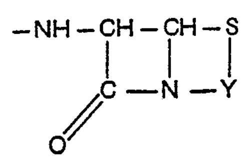

II

bedeutet, worin Y ein zweiwertiger Rest einer der folgenden allgemeinen Formeln ist:

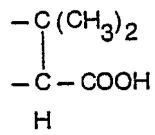 III und 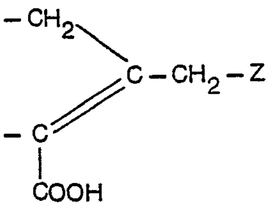 IV

worin das Kohlenstoffatom, welches die Carboxylgruppe trägt, in dem Rest Y an das Stickstoffatom gebunden ist, und Z ein Wasserstoffatom, eine Acetoxygruppe, eine Carbamoyloxygruppe, eine 5-(1-Methyl-tetrazolyl)-thio-, 5-[1-(2-Sulfoethyl)-tetrazolyl]-thio- oder eine 2-(1,3,4-Thiadiazolyl)-thiogruppe oder eine quaternäre Ammoniumgruppe, wie eine Pyridinium-, Chinolinium- oder Picoliniumgruppe, die entweder unsubstituiert oder mit einer 2-Sulfoethyl- oder Carboxymethylgruppe substituiert ist, bedeutet.

2. Imidazolcarbonsäurederivat nach Anspruch 1, worin die Aminosäure, die das Imidazolcarbonsäurederivat bildet, in der D-Form vorliegt.

3. Imidazolcarbonsäurederivat nach Anspruch 1 oder 2, wobei das Imidazolcarbonsäurederivat in der Salzform vorliegt.

## Revendications

1. Un dérivé d'acide imidazolecarboxylique répondant à la formule générale

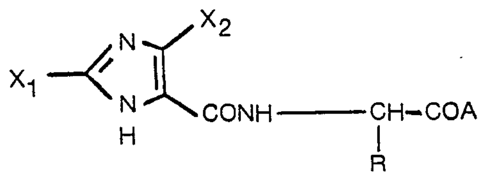

dans laquelle

$X_1$ est un hydrogène ou un groupe hydroxy, mercapto ou aralkylthio,

$X_2$ est un hydrogène, un halogène ou un groupe nitro, amino, acylamino, alcoxycarbonyle, aralcoxycarconyle ou aryloxycarbonyle, un groupe mono- ou dialkyl-, mono- ou diaralkyl-, mono- ou diarylcarbamoyle, un groupe alkyle, aralkyle ou aryle, où le nombre des atomes de carbone de l'un quelconque desdits groupes alkyles, aralkyles ou aryles est dans la gamme de 1 à 10, ou un groupe morpholinocarbonyle ou un groupe méthanesulfonylamino,

R est un groupe phényle et A est un reste de composé organique répondant à la formule générale suivante:

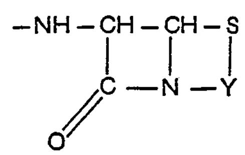

dans laquelle Y est un reste bivalent répondant à l'une des formules générales suivantes:

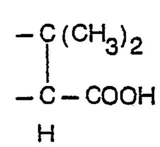 et 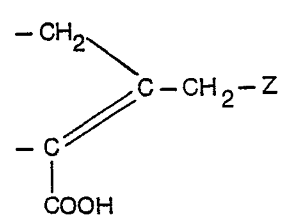

dans lesquelles l'atome de carbone qui porte le groupe carboxy est fixé à l'atome d'azote du reste Y, et Z est un atome d'hydrogène, un groupe acétoxy, un groupe carbamoyloxy, un groupe 5-(1-méthyltétrazolyl)thio, 5-[1-(2-sulfoéthyl)tétrazolyl]thio ou 2-(1,3,4-thiadiazolyl)thio ou un groupe ammonium quaternaire tel qu'un groupe pyridinium, quinolinium ou picolinium soit non substitué, soit substitué par un groupe 2-sulfoéthyle ou carboxyméthyle.

2. Un dérivé d'acide imidazolecarboxylique selon la revendication 1, dans lequel l'amino-acide qui forme ledit dérivé d'acide imidazolecarboxylique est sous la forme D.

3. Un dérivé d'acide imidazolecarboxylique selon la revendication 1 ou 2, dans lequel ledit dérivé d'acide imidazolecarboxylique est sous forme d'un sel.